# EUROPÄISCHE PATENTSCHRIFT

(11) **EP 3 510 397 B1**
(45) Veröffentlichungstag und Bekanntmachung des Hinweises auf die Patenterteilung: **07.10.2020**
(21) Anmeldenummer: 17755081.1
(22) Anmeldetag: 03.08.2017
(51) Int. Cl.: C12Q 1/6883, G01N 33/50, G01N 33/68

(54) **IN-VITRO VERFAHREN ZUR IDENTIFIZIERUNG UND ANALYSE VON SEKRETIONSPROTEINEN UNTER VERWENDUNG EINES DREIDIMENSIONALEN ZELLKULTURMODELLS DER SCHWEISSDRUESE**
IN-VITRO METHOD FOR IDENTIFYING AND ANALYSIS OF SECRETION PROTEINS USING A THREE-DIMENSIONAL CELL CULTURE MODEL OF THE PERSPIRATORY GLAND
PROCÉDÉ IN-VITRO POUR L'IDENTIFICATION ET L'ANALYSE DES PROTÉINES DE SÉCRETION À L'AIDE D'UN MODÈLE DE CULTURE DE CELLULES À TROIS DIMENSIONS DE LA GLANDE SUDORALE

(30) Priorität: 09.09.2016 DE 102016217174
(43) Veröffentlichungstag der Anmeldung: 17.07.2019
(73) Patentinhaber: Henkel AG & Co. KGaA, 40589 Düsseldorf (DE)
(72) Erfinder: KLAKA, Patricia, 51371 Leverkusen (DE); BANOWSKI, Bernhard, 40597 Düsseldorf (DE); GRÜDL, Sabine, 41812 Erkelenz (DE); WELß, Thomas, 40591 Düsseldorf (DE); GIESEN, Melanie, 47608 Geldern (DE)
(86) Internationale Anmeldenummer: PCT/EP2017/069653
(87) Internationale Veröffentlichungsnummer: WO 2018/046200

(56) Entgegenhaltungen:
- WO-A1-2005/001073
- WO-A1-2005/114178
- WO-A1-2015/175457
- CN-A- 102 091 352
- LI HAIHONG ET AL: "Three-dimensional culture and identification of human eccrine sweat glands in matrigel basement membrane matrix", CELL AND TISSUE RESEARCH, SPRINGER, DE, Bd. 354, Nr. 3, 31. August 2013 (2013-08-31), Seiten 897-902, XP035331816, ISSN: 0302-766X, DOI: 10.1007/S00441-013-1718-3 [gefunden am 2013-08-31]
- RYUICHIRO KURATA ET AL: "Isolation and Characterization of Sweat Gland Myoepithelial Cells from Human Skin", CELL STRUCTURE AND FUNCTION., Bd. 39, Nr. 2, 5. Mai 2014 (2014-05-05), Seiten 101-112, XP055411992, JP ISSN: 0386-7196, DOI: 10.1247/csf.14009
- HUANG ZHIJIAN ET AL: "Shh promotes sweat gland cell maturation in three-dimensional culture", CELL AND TISSUE BANKING, SPRINGER, NL, Bd. 17, Nr. 2, 23. Februar 2016 (2016-02-23), Seiten 317-325, XP035903320, ISSN: 1389-9333, DOI: 10.1007/S10561-016-9548-7 [gefunden am 2016-02-23]
- HUANG S ET AL: "In vitro constitution and in vivo implantation of engineered skin constructs with sweat glands", BIOMATERIALS, ELSEVIER SCIENCE PUBLISHERS BV., BARKING, GB, Bd. 31, Nr. 21, 1. Juli 2010 (2010-07-01), Seiten 5520-5525, XP027059052, ISSN: 0142-9612 [gefunden am 2010-04-15]
- KELSEA M. HUBKA ET AL: "Dissociative and Nondissociative Models for Culture of Human Eccrine Glands for Toxicology Testing and Tissue Engineering Applications", APPLIED IN VITRO TOXICOLOGY, Bd. 1, Nr. 3, 1. September 2015 (2015-09-01), Seiten 187-197, XP055399981, ISSN: 2332-1512, DOI: 10.1089/aivt.2015.0013

## Beschreibung

Die vorliegende Erfindung betrifft ein in-vitro Verfahren zur Identifizierung und Analyse von Sekretionsproteinen, bei welchem zunächst ein dreidimensionales Schweißdrüsenäquivalent mit 500 bis 500.000 Schweißdrüsenzellen sowie einem Durchmesser von 100 bis 6.000 µm bereitgestellt und anschließend eine Identifizierung und Analyse von in diesem Äquivalent vorliegenden Sekretionsproteinen erfolgt. Die erfindungsgemäß verwendeten dreidimensionalen Schweißdrüsenäquivalente weisen geordnete Strukturen sowie unterschiedlich differenzierte Zellen auf und zeigen ein Reaktionsvermögen sowohl auf Genexpressionsebene als auch auf Ebene der Proteinexpression auf einen externen Stimulus, beispielsweise einen cholinergenen Stimulus durch Acetylcholin (auch als ACh bezeichnet).

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Achselnässe und Körpergeruch entstehen durch die Sekretion aus ekkrinen und apokrinen Schweißdrüsen in der humanen Achselhöhle. Während die ekkrinen Drüsen der Thermoregulation des Körpers dienen und für die Entstehung der Achselnässe verantwortlich sind, scheiden die apokrinen Drüsen ein viskoses Sekret in Reaktion auf Stress ab, aus welchem durch bakterielle Zersetzung unangenehmer Körpergeruch entsteht.

Erste Forschungsarbeiten an nativen ekkrinen und apokrinen Schweißdrüsen wurden bereits Anfang des 20. Jahrhunderts durchgeführt, um diese zur Gruppe der exokrinen Drüsen gehörenden Hautanhangsgebilde zu klassifizieren. Schweißdrüsen lassen sich hiernach in apokrine und ekkrine Schweißdrüsen sowie eine Mischform aus apokrinen und ekkrinen Schweißdrüsen (auch als apoekkrine Schweißdrüse bezeichnet) einteilen. Die zuvor genannten Formen können anhand morphologischer und charakteristischer Merkmale unterschieden werden.

Die ekkrine Schweißdrüse, insbesondere die humane ekkrine Schweißdrüse, gehört zu den unverzweigten gewunden tubulären Drüsen und lässt sich in das sekretorische Endstück (auch als Coil bezeichnet), den dermalen Ausführgang (auch als Dukt bezeichnet) sowie den epidermalen Ausführgang (auch als Acrosyringium bezeichnet) unterteilen. Die in diesen Drüsenabschnitten vorhanden Zellen weisen unterschiedliche Aufgaben und Funktionen auf, wie beispielsweise die Sekretion im Coil, Rückresorption von Ionen im Dukt sowie Abgabe des Sekrets, insbesondere des Schweißes, an die umliegende Haut durch das Acrosyringium. Die ekkrinen Schweißdrüsen werden hauptsächlich durch den Neurotransmitter Acetylcholin (ACh) stimuliert, jedoch ist auch eine purinerge (beispielsweise mit ATP/UTP) sowie eine aß-adrenerge (beispielsweise mit Noradrenalin) Stimulation möglich.

Im Hinblick auf die Vermeidung von Achselnässe und/oder Körpergeruch ist es daher wünschenswert, die Sekretion aus ekkrinen und/oder apokrinen Schweißdrüsen zu vermindern und/oder zu vermeiden. Dies kann beispielsweise dadurch erreicht werden, dass die Ausführungsgänge der ekkrinen Schweißdrüsen mittels sogenannter Plugs verstopft. Hierzu werden im Stand der Technik schweißhemmende Aluminium- und/oder Aluminium-Zirkoniumsalze eingesetzt, welche jedoch vom Verbraucher als kritisch empfunden werden. Weiterhin werden im Stand der Technik antibakterielle Mittel eingesetzt, welche den bakteriellen Abbau des Schweißes verhindern. Derartige Mittel können jedoch die natürliche Mikroflora der Haut unter der Achselhöhle negativ beeinflussen. Es ist daher wünschenswert, kosmetische Mittel bereitzustellen, welche in der Lage sind, die Achselnässe und/oder den Körpergeruch zuverlässig zu verhindern und welche frei von Aluminium- und/oder Aluminium-Zirkoniumsalzen sowie antibakteriell wirkenden Verbindungen sind. Eine Möglichkeit zur Bereitstellung derartiger Mittel besteht in dem Einsatz von Substanzen, welche die Stimulierung und/oder die biologischen Prozesse der Schweißdrüsen effektiv hemmen und somit die Schweißsekretion vermindern bzw. vermeiden. Um derartige Substanzen identifizieren zu können, können in-vivo Tests mit Versuchsteilnehmern durchgeführt werden. Solche Tests sind jedoch aufwändig und erlauben keine Screeningverfahren mit hohen Durchsatzraten. Zum anderen können in-vitro Tests unter Einsatz von Zellmodellen von Schweißdrüsen eingesetzt werden, an welchen der Einfluss von Testsubstanzen auf die Stimulation der Schweißdrüsen untersucht werden kann.

Damit die in-vitro erhaltenen Testergebnisse gut auf die in-vivo Situation übertragbar sind, muss das eingesetzte Zellmodell der Schweißdrüse die in-vivo-Situation möglichst genau nachbilden. Hierzu sind dreidimensionale Zellmodelle erforderlich, da die im Stand der Technik bekannten zweidimensionalen Modelle keine ausreichende physiologische Nähe zur nativen Schweißdrüse aufweisen und damit die in-vivo-Situation nur unzureichend wiedergeben. Darüber hinaus ist eine Aufklärung des Schweißsekretionsmechanismus erforderlich. Denn nur auf diese Weise können sogenannte biologische Targets, insbesondere durch die Schweißdrüsenzellen produzierte Proteine, identifiziert werden, deren Beeinflussung durch Testsubstanzen zu einer verminderten Schweißprodukten führt. Ein mögliches biologisches Target, welches im Zusammenhang mit der Schweißproduktion stehen könnte, sind Sekretionsproteine, welche während der Schweißsekretion von den Schweißdrüsenzellen gebildet werden. WO2005/114178 und WO2005/001073 offenbaren die Herstellung organoider Körperchen aus Stammzellen mit Hilfe von Matrices oder Trägern.

Es besteht daher weiterhin ein Bedarf an in-vitro Verfahren, mit deren Hilfe biologische Targets, welche für eine erhöhte Schweißproduktion verantwortlich sind, identifiziert und analysiert werden können. Nach der Identifikation und Analyse derartiger Targets erfolgt bevorzugt die Untersuchung des Einflusses verschiedener Testsubstanzen auf diese Targets. Derartige in-vitro Verfahren sollen standardisierbar, kostengünstig und schnell durchführbar sein, so dass die Ermittlung des Einflusses von Testsubstanzen auf die biologischen Targets in Screeningverfahren mit hohen Durchsatzraten ermöglicht wird.

Der vorliegenden Erfindung lag daher die Aufgabe zugrunde, ein in-vitro Verfahren zur Identifikation und Analyse von Sekretionsproteinen bereitzustellen, welches standardisierbar, kostengünstig sowie schnell durchführbar ist und dessen Ergebnisse sich auf die in-vivo-Situation übertragen lassen.

Es wurde nun überraschend gefunden, dass mit Hilfe bestimmter dreidimensionaler Schweißdrüsenäquivalente eine Identifikation und Analyse von Sekretionsproteinen ermöglicht wird. Die eingesetzten dreidimensionalen Schweißdrüsenäquivalente weisen eine geordnete Struktur auf. Weiterhin bilden die primären Schweißdrüsenzellen dieser Äquivalente die gleichen Charakteristika aus wie native Schweißdrüsen. Daher lassen sich die mit diesen Äquivalenten erhaltenen Messdaten in Bezug auf die Identifikation und Analyse von Sekretionsproteinen sehr gut auf die in-vivo-Situation übertragen.

Ein erster Gegenstand der vorliegenden Erfindung ist somit ein in-vitro Verfahren zur Identifikation und Analyse von Sekretionsproteinen in der humanen Schweißdrüse, umfassend die folgenden Verfahrensschritte:
a) Bereitstellen mindestens eines dreidimensionalen Schweißdrüsenäquivalents, umfassend 500 bis 500.000 Schweißdrüsenzellen, wobei das mindestens eine dreidimensionale Schweißdrüsenäquivalent einen Durchmesser von 100 bis 6.000 µm aufweist und frei von Matrixverbindungen und Trägern ist,
b) Identifizierung und Analyse von mindestens einem Sekretionsprotein in dem in Verfahrensschritt a) bereitgestellten mindestens einen dreidimensionalen Schweissdrüsenäquivalent, wobei es sich bei dem mindestens einen Sekretionsprotein um ein antibakterielles Sekretionsprotein handelt.

Die in dem erfindungsgemäßen Verfahren verwendeten dreidimensionalen Schweißdrüsenäquivalente bilden eine geordnete Struktur und weisen differenzierte Zellen mit den gleichen Charakteristika auf wie native Schweißdrüsen. Weiterhin zeigen diese Äquivalente eine Reaktion auf Genexpressions- als auch auf Proteinexpressionsebene auf einen Stimulus durch Acetylcholin (ACh). Die mit dem erfindungsgemäßen Verfahren erhaltenen Ergebnisse lassen sich daher gut auf die in-vivo-Situation übertragen. Durch Verwendung von kultivierten primären Schweißdrüsenzellen bei der Herstellung der Äquivalente kann eine hohe Standardisierung erreicht werden, da aus den kultivierten Zellen eine Vielzahl von Äquivalenten mit gleichen Eigenschaft erzeugt werden können. Weiterhin können durch den Einsatz von kultivierten primären Schweißdrüsenzellen Äquivalente mit annährend gleichen Zahlen an Schweißdrüsenzellen erzeugt werden, was ebenfalls eine hohe Standardisierbarkeit sicherstellt.

Unter dem Begriff "Sekretionsproteine" werden erfindungsgemäß Proteine verstanden, welche von den Zellen der dreidimensionalen Schweißdrüsenäquivalente gebildet und in den Extrazellularraum ausgeschieden werden. Derartige Proteine werden in der Literatur auch als sekretorische Proteine bezeichnet.

Weiterhin wird unter einem dreidimensionalen Schweißdrüsenäquivalent erfindungsgemäß ein Zellmodell aus Schweißdrüsenzellen verstanden, welches eine Ausdehnung in alle drei Raumrichtungen aufweist und in welchem die Zellen eine ähnliche Funktion, insbesondere eine identische Funktion, wie die Zellen einer nativen Schweißdrüse zeigen.

In Verfahrensschritt a) des erfindungsgemäßen Verfahrens wird zunächst mindestens ein dreidimensionales Schweißdrüsenäquivalent mit einer bestimmten Zellzahl und einem bestimmten Durchmesser bereitgestellt.

Besonders bevorzugte dreidimensionale Schweißdrüsenäquivalente weisen bestimmte Durchmesser auf. Es ist daher erfindungsgemäß vorteilhaft, wenn das in Verfahrensschritt a) bereitgestellte mindestens eine dreidimensionale Schweißdrüsenäquivalent einen Durchmesser von 100 bis 4.000 µm, vorzugsweise von 100 bis 2.000 µm, insbesondere von 200 bis 1.500 µm, aufweist. Der Durchmesser der bevorzugt kugelförmigen erfindungsgemäß verwendeten Schweißdrüsenäquivalente kann beispielsweise mittels mikroskopischer Vermessung unter Verwendung der Software "CellSens" bestimmt werden.

Im Rahmen der vorliegenden Erfindung sind die in Verfahrensschritt a) verwendeten Schweißdrüsenäquivalente frei von Matrixverbindungen und Trägern. Unter Matrixverbindungen sind hierbei Verbindungen zu verstehen, welche zur Ausbildung räumlicher Netzwerke in der Lage sind. Hierunter fallen jedoch nicht die von den Zellen der Äquivalente selbst produzierten und ausgeschiedenen Substanzen, welche zur Ausbildung räumlicher Netzwerke in der Lage sind. Weiterhin werden unter Trägern im Sinne der vorliegenden Erfindung selbsttragende Stoffe verstanden, welche als Unterlage bzw. Gerüst für die Schweißdrüsenzellen dienen können. Gemäß der vorliegenden Erfindung ist das in Verfahrensschritt a) bereitgestellte mindestens eine dreidimensionale Schweißdrüsenäquivalent frei von Matrixverbindungen und Trägern.

Unter dem Begriff _{"}frei von" wird erfindungsgemäß verstanden, dass die dreidimensionalen Schweißdrüsenäquivalente weniger als 1 Gew.-%, bezogen auf das Gesamtgewicht des dreidimensionalen Schweißdrüsenäquivalents, an Matrixverbindungen und/oder Träger enthalten. Es ist daher im Rahmen der vorliegenden Erfindung vorteilhaft, wenn die in Verfahrensschritt a) eingesetzten dreidimensionalen Schweißdrüsenäquivalente 0 bis 1 Gew.-%, vorzugsweise 0 bis 0,5 Gew.-%, bevorzugt 0 bis 0,2 Gew.-%, insbesondere 0 Gew.-%, jeweils bezogen auf das Gesamtgewicht des dreidimensionalen Schweißdrüsenäquivalents, an Matrixverbindungen und Trägern enthalten.

Erfindungsgemäß sind die in Verfahrensschritt a) verwendeten dreidimensionalen Schweißdrüsenäquivalente frei von bestimmten Matrixverbindungen und Träger. Es ist daher bevorzugt, wenn das dreidimensionale Schweißdrüsenäquivalent keine Matrixverbindungen und/oder Träger enthält, welche ausgewählt sind aus der Gruppe von Collagenen, insbesondere Collagen Typ I und/oder Typ III und/oder Typ IV, Scleroproteinen, Gelatinen, Chitosanen, Glucosaminen, Glucosaminoglucanen (GAG), Heparinsulfatproteoglucanen, sulfatierten Glycoproteinen, Wachstumsfaktoren, vernetzten Polysacchariden, vernetzten Polypeptiden sowie deren Mischungen.

Besonders bevorzugt handelt es sich bei dem in Verfahrensschritt a) bereitgestellten dreidimensionalen Schweißdrüsenäquivalent um ein Äquivalent der ekkrinen und/oder apokrinen humanen Schweißdrüse. Bevorzugte Ausführungsformen der vorliegenden Erfindung sind daher dadurch gekennzeichnet, dass das in Verfahrensschritt a) bereitgestellte mindestens eine dreidimensionale Schweißdrüsenäquivalent ein dreidimensionales Schweißdrüsenäquivalent der ekkrinen und/oder apokrinen humanen Schweißdrüse ist. Derartige Schweißdrüsenäquivalente sind besonders gut zur Identifizierung und Analyse von Sekretionsproteinen sowie für die Ermittlung des Einflusses von Testsubstanzen auf diese Proteine geeignet.

Darüber hinaus ist es erfindungsgemäß besonders bevorzugt, wenn das in Verfahrensschritt a) bereitgestellte dreidimensionale Schweißdrüsenäquivalent aus humanen ekkrinen und/oder apokrinen Schweißdrüsen hergestellt wurde. Es ist daher im Rahmen der vorliegenden Erfindung vorteilhaft, wenn das in Verfahrensschritt a) bereitgestellte mindestens eine dreidimensionale Schweißdrüsenäquivalent ein aus ekkrinen und/oder apokrinen nativen humanen Schweißdrüsenzellen gewonnenes dreidimensionales Schweißdrüsenäquivalent ist.

Darüber hinaus hat es sich erfindungsgemäß als vorteilhaft erwiesen, wenn die in Verfahrensschritt a) bereitgestellten dreidimensionalen Schweißdrüsenäquivalente mindestens eine bestimme Zellart aufweisen. Der Einsatz derartiger Äquivalente führt zu einer besonders guten Identifizierung und Analyse von Sekretionsproteinen. Bevorzugt Ausführungsformen der vorliegenden Erfindung sind daher dadurch gekennzeichnet, dass das in Verfahrensschritt a) bereitgestellte mindestens eine dreidimensionale Schweißdrüsenäquivalent mindestens eine Zellart, ausgewählt aus der Gruppe von (i) Coilzellen, insbesondere clear cells, dark cells, sowie Myoepitheliumzellen, (ii) Duktzellen sowie (iii) deren Mischungen, enthält. Unter dem Begriff "clear cells" werden erfindungsgemäß Zellen verstanden, welche ein klares bzw. ungefärbtes Cytoplasma bei Anfärbung mit Farbstoffen, insbesondere mit Hematoxylin und Eosin aufweisen. Derartige "clear cells" sind bevorzugt sekretorische Zellen des Epitheliums, wobei die Plasmamembran an der apikalen und lateralen Oberfläche stark gefaltet ist. Das Cytoplasma dieser "clear cells" enthält hohe Mengen an Glycogen sowie viele Mitochondrien. Die Zellen befinden sich bevorzugt in Kontakt mit dem Lumen. Die wässrige Komponente des Schweißes, welche Elektrolyte und anorganische Substanzen enthält, wird bevorzugt von dieser Zellart ausgeschieden. Hingegen sind die zuvor angeführten "dark cells" Zellen, deren Vakuolen eine positive Anfärbung auf Mucopolysaccharidsäure aufweisen, deren Cytoplasma sich also durch Farbstoffe anfärben lässt. Diese "dark cells" stehen in Kontakt mit der Basalmembran und weisen im Vergleich zu den "clear cells" nur wenige Mitochondrien auf. Bevorzugt werden Makromoleküle, wie beispielsweise Glycoproteine, von diesen "dark cells" abgeschieden. Unter den zuvor angeführten "Myoepithelzellen" sind kontraktile Epithelzellen zu verstehen, welche ein Zytosklett mit sogenannten Gap junctions aufweisen und sich daher kontrahieren können. Auf diese Weise wird die Sekretabgabe aus den Drüsenendstücken unterstützt. Derartige Zellen befinden sich bevorzugt zwischen der Basalmembran und den zuvor angeführten "clear cells" und "dark cells". Schließlich werden unter dem Begriff "Duktzellen" erfindungsgemäß Zellen verstanden, welche die Wand des Duktes bilden und ein stratifiziertes kubisches Epithel aufweisen. Die zuvor angeführten Zellarten können durch neben der Verwendung von Hematoxylin und Eosin auch mittels immunzytochemischen Färbungen unter Einsatz von für diese Zellen spezifische Marker bestimmt werden. Ein für Myoepithelzellen einsetzbarer spezifischer Marker ist alpha-smooth muscle actin (auch als α-SMA bezeichnet). Als spezifische Marker "clear cells" eignet sich beispielsweise Substance P sowie S100. Weiterhin kann für "dark cells" der unter der Bezeichnung CGRP (calcitonin-gene related peptide) bekannte Marker, für Duktzellen die spezifischen Marker Cytokeratin 10 (auch als CK10 bezeichnet) und CD200 verwendet werden.

Im Folgenden sind besonders bevorzugte in Verfahrensschritt a) verwendete dreidimensionale Schweißdrüsenäquivalente beschrieben.

Eine besonders bevorzugte Ausführungsform dieses Erfindungsgegenstands ist demnach die Bereitstellung eines dreidimensionalen Schweißdrüsenäquivalents der ekkrinen und/oder apokrinen humanen Schweißdrüse, umfassend 500 bis 500.000 Schweißdrüsenzellen, wobei das dreidimensionale Schweißdrüsenäquivalent einen Durchmesser von 200 bis 1.500 µm aufweist.

Weiterhin ist eine besonders bevorzugte Ausführungsform dieses Erfindungsgegenstands die Bereitstellung eines aus ekkrinen und/oder apokrinen nativen humanen Schweißdrüsenzellen gewonnenes dreidimensionales Schweißdrüsenäquivalent, umfassend 500 bis 500.000 Schweißdrüsenzellen, wobei das dreidimensionale Schweißdrüsenäquivalent einen Durchmesser von 200 bis 1.500 µm aufweist.

Darüber hinaus ist eine besonders bevorzugte Ausführungsform dieses Erfindungsgegenstands die Bereitstellung eines dreidimensionalen Schweißdrüsenäquivalents, umfassend 500 bis 500.000 Schweißdrüsenzellen, wobei das dreidimensionale Schweißdrüsenäquivalent einen Durchmesser von 200 bis 1.500 µm aufweist und mindestens eine Zellart, ausgewählt aus der Gruppe von clear cells, dark cells, Myoepitheliumzellen, Duktzellen sowie deren Mischungen enthält.

Zudem ist eine besonders bevorzugte Ausführungsform dieses Erfindungsgegenstands die Bereitstellung eines aus ekkrinen und/oder apokrinen nativen humanen Schweißdrüsenzellen gewonnenes dreidimensionales Schweißdrüsenäquivalent, umfassend 500 bis 500.000 Schweißdrüsenzellen, wobei das dreidimensionale Schweißdrüsenäquivalent einen Durchmesser von 200 bis 1.500 µm aufweist und mindestens eine Zellart, ausgewählt aus der Gruppe von clear cells, dark cells, Myoepitheliumzellen, Duktzellen sowie deren Mischungen enthält.

Weiterhin ist eine besonders bevorzugte Ausführungsform dieses Erfindungsgegenstands die Bereitstellung eines dreidimensionalen Schweißdrüsenäquivalents der ekkrinen und/oder apokrinen humanen Schweißdrüse, umfassend 500 bis 500.000 Schweißdrüsenzellen, wobei das dreidimensionale Schweißdrüsenäquivalent einen Durchmesser von 200 bis 1.500 µm aufweist und 0 Gew.-%, bezogen auf das Gesamtgewicht des dreidimensionalen Schweißdrüsenäquivalents, an Matrixverbindungen und Trägern enthält.

Zudem ist eine besonders bevorzugte Ausführungsform dieses Erfindungsgegenstands die Bereitstellung eines dreidimensionalen Schweißdrüsenäquivalents der ekkrinen und/oder apokrinen humanen Schweißdrüse, umfassend 500 bis 500.000 Schweißdrüsenzellen, wobei das dreidimensionale Schweißdrüsenäquivalent einen Durchmesser von 200 bis 1.500 µm aufweist und 0 Gew.-%, bezogen auf das Gesamtgewicht des dreidimensionalen Schweißdrüsenäquivalents, an Matrixverbindungen und Trägern enthält, wobei die Matrixverbindungen und/oder Träger sind aus der Gruppe von Collagenen, insbesondere Collagen Typ I und/oder Typ III und/oder Typ IV, Scleroproteinen, Gelatinen, Chitosanen, Glucosaminen, Glucosaminoglucanen (GAG), Heparinsulfatproteoglucanen, sulfatierten Glycoproteinen, Wachstumsfaktoren, vernetzten Polysacchariden, vernetzten Polypeptiden sowie deren Mischungen.

Die in dem Verfahrensschritt a) bereitgestellten dreidimensionalen Schweißdrüsenäquivalente weisen eine höhere Standardisierbarkeit und Verfügbarkeit auf wie isolierte Schweißdrüsen und besitzen eine größere Nähe zur in-vivo Situation als eindimensionale und zweidimensionale Schweißdrüsenmodelle. Weiterhin stellen diese Äquivalente eine kostengünstige Alternative zu in-vivo Studien am Menschen dar, da mittels dieser Äquivalente Sekretionsproteine identifiziert sowie deren Einfluss auf die Schweißsekretion analysiert werden können. Denn die dreidimensionalen Schweißdrüsenäquivalente simulieren die Schweißdrüse in-vivo sowohl in ihrer Struktur als auch in ihrer histologischen Zusammensetzung, so dass die mit diesen Äquivalenten erhaltenen Informationen gut auf den Menschen übertragbar sind.

Die in Verfahrensschritt a) bereitgestellten dreidimensionalen Schweißdrüsenäquivalente lassen sich beispielsweise durch das folgende Herstellungsverfahren erhalten.

In einem ersten Schritt werden zunächst isolierte Schweißdrüsen bereitgestellt, welche aus Hautbiopsien oder dergleichen erhalten werden können und welche aus ihrer natürlichen Umgebung entfernt wurden. Bevorzugt werden die isolierten Schweißdrüsen des ersten Schritts durch Isolierung nativer Schweißdrüsen, insbesondere nativer ekkriner und/oder apokriner Schweißdrüsen, aus der menschlichen Haut erhalten, wobei die Isolierung der nativen Schweißdrüsen bevorzugt durch enzymatischen Verdau der menschlichen Haut unter Verwendung einer Mischung aus 2 bis 3 mg/ml Collagenase II und 0,1 bis 0,2 mg/ml Thermolysin für 3 bis 6 Stunden bei 35 bis 40 °C, insbesondere bei 37°C, erfolgt.

Diese isolierten Schweißdrüsen werden in einem zweiten Schritt in einem bestimmten Nährmedium kultiviert, um eine Zellkultur zu erhalten. Eine besonders gute Kultivierung der in dem ersten Schritt erhaltenen isolierten Schweißdrüsenzellenwird erreicht, wenn eine Mischung aus DMEM und Ham's F12 im Gewichtsverhältnis 3:1, welche zusätzlich 10 Gew.-%, bezogen auf das Gesamtgewicht der Mischung, fetales Kälber Serum (FCS), enthält, als Nährmedium verwendet wird. Die Kultivierung dieser Zellen in dem zuvor beschriebenen Nährmedium erfolgt vorzugsweise für 7 bis 28 Tage, insbesondere für 14 Tage, bei einer Temperatur von 36 bis 38 °C und einem CO₂-Gehalt von 5 Gew.-%, bezogen auf das Gesamtgewicht der zur Kultivierung eingesetzten Atmosphäre.

Aus den kultivierten Zellen wird im dritten Schritt eine Zellpräparation von primären Schweißdrüsenzellen in einem Nährmedium hergestellt, wobei die Zellzahl der primären Schweißdrüsenzellen in der Zellpräparation 50 bis 250.00 Zellen pro µL, vorzugsweise 100 bis 10.000 Zellen pro µL, bevorzugt 150 bis 5.000 Zellen pro µL, weiter bevorzugt 200 bis 3.200 Zellen pro µL, noch weiter bevorzugt 300 bis 1.000 Zellen pro µL, insbesondere 400 bis 600 Zellen pro µL Nährmedium beträgt. Die Zellpräparation von primären Schweißdrüsenzellen wird bevorzugt durch Ablösung der im zweiten Schritt kultivierten Schweißdrüsenzellen, insbesondere durch schonende Trypsinierung, Kultivierung dieser abgelösten Schweißdrüsenzellen in Monolayer-Kulturen, Suspendierung der kultivierten primären Schweißdrüsenzellen in einem Nährmedium sowie Einstellung der Zellzahl hergestellt. Für die Kultivierung der abgelösten Schweißdrüsenzellen sowie zur Herstellung der Zellsuspension hat es sich als vorteilhaft erwiesen, wenn eine Mischung aus DMEM und Ham's F12 im Gewichtsverhältnis 3:1, welche zusätzlich 10 Gew.-%, bezogen auf das Gesamtgewicht der Mischung, fetales Kälber Serum (FCS), enthält, als Nährmedium verwendet wird. Die Kultivierung der abgelösten Schweißdrüsenzellen wird vorzugsweise bei einer Temperatur von 36 bis 38 °C und einem CO₂-Gehalt von 5 Gew.-%, bezogen auf das Gesamtgewicht der zur Kultivierung eingesetzten Atmosphäre, bis zur Kofluenz durchgeführt.

Anschließend werden in einem vierten Schritt 10 bis 100 µL, vorzugsweise von 20 bis 80 µL, bevorzugt von 30 bis 70 µL, insbesondere von 40 bis 60 µL dieser Zellpräparation in hängenden Zustand, also in Form eines von einer Oberfläche frei schwebend herabhängenden Tropfens, kultiviert, bis sich die dreidimensionalen Schweißdrüsenäquivalente ausgebildet haben. In diesem Zusammenhang hat sich die Verwendung sogenannter Hanging-Drop-Wells, wie beispielsweise in der Offenlegungsschrift WO 2012/014047 A1 offenbart und kommerziell von der Firma Insphero als GravityPLUS®-Einsaatplatte mit SureDrop® Inlet-Einführsystem sowie GravityTRAP®-Ernteplatte erhältlich, als vorteilhaft erwiesen. Bevorzugt erfolgt die Kultivierung der Zellpräparation in hängendem Zustand für einen Zeitraum von 1 bis 25 Tagen, insbesondere von 2 bis 7 Tagen, bei einer Temperatur von 36 bis 38 °C und einem CO₂-Gehalt von 5 Gew.-%, bezogen auf das Gesamtgewicht der zur Kultivierung eingesetzten Atmosphäre. Hierbei ist es bevorzugt, wenn während der Kultivierungsdauer, insbesondere nach 1 bis 3 Tagen, 40 Volumenprozent, bezogen auf das Gesamtvolumen der zuvor angeführten Zellpräparation, des Nährmediums der Zellpräparation durch frisches Nährmedium ersetzt werden.

Nach Isolation der erhaltenen Äquivalente durch Zugabe von 50 bis 200 µL, insbesondere von 70 bis 100 µL, Nährmedium können die Äquivalente direkt für den Verfahrensschritt b) des erfindungsgemäßen Verfahrens verwendet oder erneut kultiviert werden. Die erneute Kultivierung der erhaltenen Äquivalente erfolgt bevorzugt für einen Zeitraum von 1 bis 6 Tagen bei einer Temperatur von 36 bis 38 °C und einem CO₂-Gehalt von 5 Gew.-%, bezogen auf das Gesamtgewicht der zur Kultivierung eingesetzten Atmosphäre.

Die Bereitstellung der dreidimensionalen Schweißdrüsenäquivalente in Verfahrensschritt a) erfolgt daher besonders bevorzugt mit den nachfolgend beschriebenen Verfahren, welches die folgenden Schritte in der angegebenen Reihenfolge umfasst:
(i) Bereitstellung von isolierten Schweißdrüsen, wobei die isolierten Schweißdrüsen durch Isolierung nativer ekkriner und/oder apokriner Schweißdrüsen aus der menschlichen Haut und anschließender Suspendierung dieser isolierten Schweißdrüsen in Nährmedium erhalten werden,
(ii) Bereitstellung einer Zellpräparation von primären Schweißdrüsenzellen aus den in Verfahrensschritt (i) isolierten Schweißdrüsen, wobei die Zellzahl der primären Schweißdrüsenzellen in der Zellpräparation 400 bis 600 Zellen pro µL beträgt und wobei die Zellpräparation von primären Schweißdrüsenzellen ein Volumen von 40 bis 60 µL aufweist,
(iii) Kultivierung der in Verfahrensschritt (ii) bereitgestellten Zellpräparation in einem hängenden Zustand, wobei der hängende Zustand der Zellpräparation durch Verwendung einer Hanging-Drop-Multiwellplatte erreicht wird und wobei während der Kultivierungsdauer 40 Volumenprozent, bezogen auf das Gesamtvolumen der in diesem Verfahrensschritt verwendeten Zellpräparation, des Nährmediums der Zellpräparation durch frisches Nährmedium ersetzt werden,
(iv) Isolierung des in Verfahrensschritts (iii) erhaltenen dreidimensionalen Schweißdrüsenäquivalents, wobei die Isolierung des dreidimensionalen Schweißdrüsenäquivalents durch Zugabe von 50 bis 200 µL Nährmedium zur Ablösung des Modells erfolgt,
(v) Gegebenenfalls Kultivierung des in Verfahrensschritt (iv) isolierten dreidimensionalen Schweißdrüsenäquivalents für einen Zeitraum von 1 bis 6 Tagen bei einer Temperatur von 36 bis 38 °C und einem CO₂-Gehalt von 5 Gew.-%, bezogen auf das Gesamtgewicht der zur Kultivierung eingesetzten Atmosphäre.

Da im Rahmen der vorliegenden Erfindung bevorzugt die Identifikation und Analyse von Sekretionsproteinen der ekkrinen und/oder apokrinen humanen Schweißdrüse erfolgen soll, erfolgt die Herstellung der in Schritt a) bereitgestellten Äquivalente unter Verwendung von ekkrinen und/oder apokrinen nativen humanen Schweißdrüsen. Unter ekkrinen und/oder apokrinen nativen Schweißdrüsen sind hierbei ekkrine und/oder apokrine Schweißdrüsen zu verstehen, welche aus der Haut des Menschen, insbesondere aus Hautbiopsien des Menschen oder durch andere Verfahren, isoliert wurden.

Weiterhin werden die in Schritt a) bereitgestellten dreidimensionalen Schweißdrüsenäquivalente bevorzugt ausschließlich unter Verwendung von in-vitro Methoden hergestellt. Folglich sind keine Verfahrensschritte enthalten, bei welchen in-vivo Methoden eingesetzt werden. Somit können diese Äquivalente auch zur Testung von Substanzen verwendet werden, welche zur kosmetischen Anwendung vorgesehen sind. Weiterhin erlaubt dieses Herstellungsverfahren eine kostengünstige Herstellung von standardisierten Äquivalenten, welche in Screeningverfahren mit hohen Durchsatzraten eingesetzt werden können. Zudem resultiert dieses Herstellverfahren in dreidimensionalen Schweißdrüsenäquivalenten, welche geordnete Strukturen ausbilden, unterschiedlich differenzierte Zellen aufweisen und Schweißdrüsenspezifische Marker exprimieren, so dass eine gute Übertragbarkeit von in-vitro Daten auf die in-vivo-Situation ermöglicht wird.

Ein Herstellverfahren für die in Verfahrensschritt a) des erfindungsgemäßen Verfahrens bereitgestellten dreidimensionalen Schweißdrüsenäquivalente ist beispielsweise in der deutschen Anmeldung DE 10 2015 222 279 offenbart, auf deren Inhalt hiermit vollinhaltlich Bezug genommen wird.

In dem zweiten Verfahrensschritt des erfindungsgemäßen Verfahrens erfolgt die Identifizierung und Analyse mindestens eines antibakteriellen Sekretionsproteins in dem in Verfahrensschritt a) bereitgestellten dreidimensionalen Schweißdrüsenäquivalent.

Erfindungsgemäße biologische Targets stellen antimikrobiell wirkende Sekretionsproteine dar. Es ist daher erfindungsgemäß wenn es sich bei dem mindestens einen Sekretionsprotein in Verfahrensschritt b) um ein antibakterielles Sekretionsprotein handelt.

Im Rahmen der vorliegenden Erfindung werden bestimmte Sekretionsproteine, insbesondere bestimmte antibakteriell wirkende Sekretionsproteine, identifiziert und analysiert. Die vorliegende Erfindung ist daher dadurch gekennzeichnet, dass das mindestens eine antibakterielle Sektretionsprotein, in Verfahrensschritt b) ausgewählt ist aus der Gruppe von Mucinen, Dermicidinen, Beta-Defensinen, Secretoglobinen, Lysozym, Albumin, Laktat-Dehydrogenase (LDH), Galanin, Prolactin-induziertes Protein (PIP), Vasoactives Intestinalpeptid (VIP), Zink-Alpha-2-Glykoprotein (ZA2G), Lipophilin, Apolipoproteinen, S100 Proteinen, Calcium-dependent activator protein for secretion (CADPS), Substanz P, Calcitonin-gene related peptide (CGRP) sowie deren Mischungen. Unter Mucinen werden erfindungsgemäß Proteine bezeichnet, welche zu den Proteoglycanen gehören und daher etwa 40 bis 80 Gew.-%, bezogen auf das Gesamtgewicht des Mucins, Kohlenhydrate enthalten. Weiterhin besteht ihr Proteinanteil bevorzugt aus bis zu 50 Gew.-%, bezogen auf die Gesamtmenge an Protein, aus den Aminosäuren Serin und Threonin und/oder Prolin. Sie weisen zudem ein hohes Molekulargewicht sowie eine hohe negative Ladung durch Sialyl- oder Sulfatgruppen auf. Durch diesen anionischen Charakter sowie die Anwesenheit von Hydroxgruppen innerhalb der Mucine sind diese in der Lage, eine hohe Menge an Wasser zu binden und gelartige Netzwerke auszubilden. Bei Dermicidinen, welche auch als proteolysis-inducing factor (PIF) bezeichnet werden, handelt es sich um antimikrobiell wirkende Proteine, welche aus humanen ekkrinen Schweißdrüsen ausgeschieden werden. Durch Zersetzung dieses Proteins mit Hilfe von Proteasen kann die antimikrobielle Wirkung weitergehend gesteigert werden. Unter beta-Defensinen werden kationische Proteine mit einem mittleren Molekulargewicht von 2 bis 6 kDa verstanden, welche eine antimikrobielle Wirkung gegen gram-negative und gram-positive Bakterien, Pilze und Viren aufweisen. Diese Proteine weisen drei intramolekulare Disulfidbrücken auf und werden anhand ihrer Größe und Muster ihrer Disulfidbindungen in alpha-, beta- und theta-Defensine eingeteilt. Bei Sekretoglobinen handelt es sich um kleine Proteindimere, welche über Disulfidbrücken miteinander verbunden sind und ausschließlich in Säugetieren vorkommen. Zu der Familie der Sekretoglobine zählen beispielsweise Uteroglobin, Uteroglobin-related protein 1, Uteroglobin-related peptide 2, Mammaglobin-A sowie Mammaglobin-B. Unter Apolipidoproteinen wird der Proteinanteil der Lipidproteine, beispielsweise Chylomikronen, VLDL, LDL, IDL und HDL, bezeichnet, welcher die wasserunlöslichen Lipide im Blut transportiert. Bei S100 Proteinen handelt es sich um Calciumbindende Proteine mit einer Molekülmasse von 8 bis 14 kDa, welche eine Vielzahl von zellulären Prozessen beeinflussen.

Die zuvor genannten Proteine können von den Schweißdrüsenzellen der dreidimensionalen Schweißdrüsenäquivalente ausgeschieden werden, wobei deren Ausscheidung abhängig von der Schweißproduktion ist. Daher eignen sich diese Proteine besonders als biologische Targets zur Untersuchung des Sekretionsmechanismus.

Die Identifizierung und Analyse des Sekretionsproteins, insbesondere der zuvor angeführten Proteine, wird erfindungsgemäß unter Verwendung bestimmter Methoden durchgeführt. Es ist daher erfindungsgemäß bevorzugt, wenn die Identifizierung und Analyse in Verfahrensschritt b) mit Methoden, ausgewählt aus der Gruppe von molekularbiologischen Methoden, Proteinanalysen, Assays zur Bestimmung der Funktionalität sowie deren Kombinationen erfolgt. Im Rahmen der vorliegenden Erfindung einsetzbare molekularbiologische Methoden sind beispielsweise die NGS (next generation sequencing)-Analyse sowie die qRT-PCR (quantitative real-time PCR). Mittels dieser Methoden können die zuvor angeführten Proteine mittels Genexpressionsanalysen identifiziert und quantitativ bestimmt werden. Die in den dreidimensionalen Schweißdrüsenäquivalenten erhaltenen Expressionslevel der Proteine wurden mit dem Expressionslevel dieser Proteine in Proben der humanen Schweißdrüse sowie in Proben der Vollhaut verglichen. Das Expressionslevel dieser Proteine in den dreidimensionalen Schweißdrüsenäquivalenten sowie in der humanen Schweißdrüse war signifikant höher als in den Proben der Vollhaut, so dass diese Proteine daher spezifische Markerproteine für die Schweißdrüse darstellen können. Weiterhin war die erhaltene Expression dieser Proteine in den dreidimensionalen Schweißdrüsenäquivalenten vergleichbar mit der Expression dieser Proteine in der humanen Schweißdrüsen. Die in dem erfindungsgemäßen Verfahren eingesetzten Schweißdrüsenäquivalente bilden daher die Situation in-vivo hervorragend ab und gewährleisten auf diese Weise eine gute Übertragbarkeit der in-vitro-Ergebnisse auf die in-vivo-Situation.

Geeignete Proteinanalysen sind beispielsweise Immunmarkierungen der zuvor angeführten Proteine mittels spezifischer Marker, wie die Methode der Immunfluoreszenz, Western Blot-Analysen und/oder ELISA. Mit den beiden zuletzt genannten Methoden ist ebenfalls eine quantitative Bestimmung der zuvor genannten Proteine möglich.

Im Rahmen des erfindungsgemäßen Verfahrens hat es sich als vorteilhaft erwiesen, wenn nach dem Verfahrensschritt b) ein weiterer Verfahrensschritt c) durchgeführt wird. In diesem Verfahrensschritt c) wird der Einfluss von verschiedenen Testsubstanzen auf die in Verfahrensschritt b) identifizierten Sekretionsproteine, insbesondere die zuvor angeführten bestimmten Proteine, ermittelt. Bevorzugte Ausführungsformen der vorliegenden Erfindung sind daher dadurch gekennzeichnet, dass in einem zusätzlichen Verfahrensschritt c) der Einfluss von Verbindungen auf die in Verfahrensschritt b) identifizierten Sekretionsproteine untersucht wird. Bei denen in Verfahrensschritt c) eingesetzte Verbindungen handelt es sich bevorzugt um Inhibitoren dieser Proteine, falls die Proteine die Schweißsekretion erhöhen. Vermindern die zuvor genannten Proteine jedoch die Schweißsekretion, werden als Verbindungen in Verfahrensschritt c) bevorzugt Aktivatoren eingesetzt.

In diesem Zusammenhang ist es bevorzugt, wenn in Verfahrensschritt c) bestimmte Methoden zur Ermittlung des Einflusses der Verbindung auf die in Verfahrensschritt b) identifizierten Proteine eingesetzt werden. Es ist daher erfindungsgemäße vorteilhaft, wenn der Einfluss der mindestens einen Verbindung in Verfahrensschritt c) mit Methoden, ausgewählt aus der Gruppe von molekularbiologischen Methoden, Proteinanalysen, Assays zur Bestimmung der Funktionalität sowie deren Kombinationen erfolgt. Bezüglich der Methoden wird auf die vorstehend genannten und in Verfahrensschritt b) verwendeten Methoden verwiesen, welche für die Durchführung des Verfahrensschritts c) gleichermaßen herangezogen werden können.

Die folgenden Beispiele erläutern die vorliegende Erfindung, ohne sie jedoch darauf einzuschränken:

### Beispiele:

### 1 Bereitstellung der dreidimensionalen Schweißdrüsenäquivalente (Verfahrensschritt a))

### 1.1 Isolation der Schweißdrüsen

Die nativen Schweißdrüsen wurden aus humanen Gewebeproben, sogenannten Biopsien gewonnen, welche aus plastischen Operationen von Patienten stammen, die der Verwendung des Materials zu Forschungszwecken zugestimmt haben. Das verwendete Gewebe wurde bei Oberarmstraffungen und Gesichtsstraffungen entnommen. Hieraus wurden die ekkrinen und apokrinen Schweißdrüsen aus dem Achselbereich isoliert.

Hierzu wurde die jeweilige Biopsie in kleine Stücke zerteilt und danach in Stücke von maximal ca. 1 cm x 1 cm geschnitten wurde. Anschließend erfolgte der Verdau der Haut mit einem Gemisch aus gleichen Teilen Collagenase II (5 mg/ml) und Thermolysin (0,25 mg/ml) bei 37 °C im Brutschrank für ca. 3,5 bis 5 Stunden, bis das Bindegewebe fast vollständig verdaut war. Diese Mischung wurde anschließend bei 1200 rpm für 5 Minuten zentrifugiert und der Überstand verworfen, um die Enzymlösung sowie das überschüssige Fett zu entfernen. Das entstandene Pellet wurde in DMEM-Lösung aufgenommen und die Lösung in eine Petrischale überführt. Anhand einer Mikrokapillare wurden intakte Schweißdrüsen unter einem Binokular isoliert und in frisches DMEM-Medium überführt.

### 1.2 Kultivieren der isolierten nativen Schweißdrüsen

Die in Schritt 1.1 isolierten Schweißdrüsen wurden in Collagen-I beschichtete Kulturflaschen gegeben und anschließend 25 ml Nährmedium hinzugefügt. Nach Kultivierung für 7 bis 21 Tage im Brutschrank bei 37 °C und 5 % CO₂ wurden die auswachsenden Schweißdrüsenzellen abgelöst und auf Collagen-I beschichteten Kulturflaschen erneut bis zur Konfluenz kultiviert (Monolayer-Kultur der primären Schweißdrüsenzellen).

Die Zusammensetzung des eingesetzten Nährmediums ist wie folgt:

| Bestandteile des Mediums | |
|---|---|
| DMEM/ Ham's F12 Nutrient Mix | 3 : 1 |
| Fätales Kälber Serum (FCS) | 10 % |
| EGF | 10 ng/ml |
| Hydrocortison | 0,4 µg/ml |
| Insulin | 0,12 Ul/ml |
| Choleratoxin | 10⁻¹⁰ M |
| Adenin | 2,43 g/ml |
| Gentamicin | 25 µg/ml |
| Penicillin G | 100 Ul/ml |
| Triiodothyronin | 2*10⁻⁹ M |
| Ascorbyl-2-phosphat | 1 mM |

### 1.3 Herstellung der Zellpräparation und der dreidimensionalen Schweißdrüsenäquivalente

Nach Bestimmung der genauen Zellzahlen der obigen Monolayer-Kulturen der primären Schweißdrüsenzellen wurden diese unter Verwendung des obigen Nährmediums auf eine Zellzahl von 10 bis 5.000 Zellen pro µl eingestellt und anschließend je 50 µl dieser Zellsuspension mittels des "SureDrop® Inlet"-Einführsystems in Wells einer "GravityPLUS®"-Einsaatplatte (jeweils Firma Inshpero AG, Schweiz) überführt. Die Kultivierung erfolgt bei 36 bis 38 °C und einem CO₂-Gehalt von 5 Gew.-%, bezogen auf das Gesamtgewicht der zur Kultivierung eingesetzten Atmosphäre. Nach 1 bis 3 Tagen werden jeweils 40 Vol.-% des Mediums in den Wells der "GravityPLUS®"-Einsaatplatte durch frisches Nährmedium ersetzt. Nach 2 bis 7 Tagen Kultivierung werden die 3D-Schweißdrüsenäquivalente durch Zugabe von 50 bis 200 µL Nährmedium geerntet und in eine "GravityTRAP®"-Platte (Firma Insphero AG, Schweiz) überführt. Vor der Ernte wird die "GravityTRAP®"-Platte mit 60 bis 100 µL Keratinozyten-Medium mithilfe einer Multikanalpipette angefeuchtet, um die Bildung von Luftblasen zu minimieren und den Verlust der dreidimensionalen Schweißdrüsenäquivalente zu vermeiden. Nach der Ernte wird die Platte für 1 bis 5 Minuten bei 100 bis 300 xg zentrifugiert, um Luftblasen zu entfernen. Ein Teil der dreidimensionalen Schweißdrüsenäquivalente wurde analysiert, wohingegen ein weiterer Teil für weitere 1 bis 6 Tage in den Vertiefungen der Ernteplatte bei 37 °C und 5 Gew.-% CO₂, bezogen auf das Gesamtgewicht der zur Kultivierung eingesetzten Atmosphäre, kultiviert wurde.

### 2. Identifizierung und Analyse eines Sekretionsproteins (Verfahrensschritt b))

Mittels NGS-Analysen konnte die Genexpression der zuvor angeführte besonders bevorzugten Sekretionsproteine in den dreidimensionalen Schweißdrüsenäquivalenten nachgewiesen werden. Hierzu wurde das Verfahren RNA-Seq (auch als "Gesamt-Transkriptom-Shotgun-Sequenzierung" bezeichnet) der Firma Illumina verwendet. In diesem Verfahren wird die RNA in cDNA übersetzt und anschließend die Methode der DNA-Sequenzierung angewendet. Hierfür wird die mRNA zunächst von der rRNA, bevorzugt unter Verwendung sogenannter "Ribosomal Depletion Kits", getrennt und anschließend fragmentiert, wobei die Fragmentierung sowohl vor als auch nach der Konvertierung in die cDNA erfolgen kann. Anschließend erfolgt die Sequenzierung, wobei der Einbau eines einzelnen Nucleotids in die cDNA in ein elektrisches Signal umgewandelt wird. Diese Sequenzierung kann beispielsweise mittels des Illumina Genome Analyzer II unter Durchführung der Schritte Fragmentierung der cDNA, Reinigung, Reparatur der Fragmentenden, Ligation der Adapter an die Probe, Auftrennung der Proben mit einem Agarose-Gel anhand ihrer Größe, PCR sowie Reinigung und Sequenzierung erfolgen. Nach der Sequenzierung erfolgt das Read Mapping, worin die gelesenen Fragmente (auch als Reads bezeichnet), dem Referenzgenom zugeordnet werden. Darüber hinaus konnten Substanz P, S100 Proteine und Calcitonin-gene related peptide (CGRP) auch mithilfe von Immunofluoreszenzfärbungen nachgewiesen werden.

## Patentansprüche

1. In-vitro Verfahren zur Identifikation und Analyse von Sekretionsproteinen in der humanen Schweißdrüse, umfassend die folgenden Verfahrensschritte:
a) Bereitstellen mindestens eines dreidimensionalen Schweißdrüsenäquivalents, umfassend 500 bis 500.000 Schweißdrüsenzellen, wobei das mindestens eine dreidimensionale Schweißdrüsenäquivalent einen Durchmesser von 100 bis 6.000 µm aufweist und frei von Matrixverbindungen und Trägern ist, und
b) Identifizierung und Analyse von mindestens einem Sekretionsprotein in dem in Verfahrensschritt a) bereitgestellten mindestens einen dreidimensionalen Schweißdrüsenäquivalent, wobei es sich bei dem mindestens einen Sekretionsprotein um ein antibakterielles Sekretionsprotein handelt.

2. Verfahren nach Anspruch 1, **dadurch gekennzeichnet, dass** das in Verfahrensschritt a) bereitgestellte mindestens eine dreidimensionale Schweißdrüsenäquivalent einen Durchmesser von 100 bis 4.000 µm, vorzugsweise von 100 bis 2.000 µm, insbesondere von 200 bis 1.500 µm, aufweist.

3. Verfahren nach Anspruch 1 oder 2, **dadurch gekennzeichnet, dass** die Matrixverbindungen und/oder Träger ausgewählt sind aus der Gruppe von Collagenen, insbesondere Collagen Typ I und/oder Typ III und/oder Typ IV, Scleroproteinen, Gelatinen, Chitosanen, Glucosaminen, Glucosaminoglucanen (GAG), Heparinsulfatproteoglucanen, sulfatierten Glycoproteinen, Wachstumsfaktoren, vernetzten Polysacchariden, vernetzten Polypeptiden sowie deren Mischungen.

4. Verfahren nach einem der vorangehenden Ansprüche, **dadurch gekennzeichnet, dass** das in Verfahrensschritt a) bereitgestellte mindestens eine dreidimensionale Schweißdrüsenäquivalent mindestens eine Zellart, ausgewählt aus der Gruppe von (i) Coilzellen, insbesondere clear cells, dark cells, sowie Myoepitheliumzellen, (ii) Duktzellen sowie (iii) deren Mischungen, enthält.

5. Verfahren nach einem der vorangehenden Ansprüche, **dadurch gekennzeichnet, dass** das mindestens eine Sekretionsprotein, insbesondere das mindestens eine antibakterielle Sektretionsprotein, in Verfahrensschritt b) ausgewählt ist aus der Gruppe von Mucinen, Dermicidinen, Beta-Defensinen, Secretoglobinen, Lysozym, Albumin, Laktat-Dehydrogenase (LDH), Galanin, Prolactin-induziertes Protein (PIP), Vasoactives Intestinalpeptid (VIP), Zink-Alpha-2-Glykoprotein (ZA2G), Lipophilin, Apolipoproteinen, S100 Proteinen, Calcium-dependent activator protein for secretion (CADPS), Substanz P, Calcitonin-gene related peptide (CGRP) sowie deren Mischungen.

6. Verfahren nach einem der vorangehenden Ansprüche, **dadurch gekennzeichnet, dass** die Identifizierung und Analyse in Verfahrensschritt b) mit Methoden, ausgewählt aus der Gruppe von molekularbiologischen Methoden, Proteinanalysen, Assays zur Bestimmung der Funktionalität sowie deren Kombinationen erfolgt.

7. Verfahren nach einem der vorangehenden Ansprüche, **dadurch gekennzeichnet, dass** in einem zusätzlichen Verfahrensschritt c) der Einfluss von mindestens einer Verbindung auf das in Verfahrensschritt b) identifizierte mindestens eine Sekretionsprotein untersucht wird.

8. Verfahren nach Anspruch 7, **dadurch gekennzeichnet, dass** der Einfluss der mindestens einen Verbindung in Verfahrensschritt c) mit Methoden, ausgewählt aus der Gruppe von molekularbiologischen Methoden, Proteinanalysen, Assays zur Bestimmung der Funktionalität sowie deren Kombinationen erfolgt.

## Claims

1. An in vitro method for identifying and analyzing secretion proteins in the human sweat gland, comprising the following method steps:
a) providing at least one three-dimensional sweat gland equivalent, comprising 500 to 500,000 sweat gland cells, wherein the at least one three-dimensional sweat gland equivalent has a diameter of from 100 to 6,000 µm and is free of matrix compounds and carriers, and
b) identifying and analyzing at least one secretion protein in the at least one three-dimensional sweat gland equivalent provided in method step a), wherein the at least one secretion protein is an antibacterial secretion protein.

2. The method according to claim 1, **characterized in that** the at least one three-dimensional sweat gland equivalent provided in method step a) has a diameter of from 100 to 4,000 µm, preferably from 100 to 2,000 µm, in particular from 200 to 1,500 µm.

3. The method according to claim 1 or 2, **characterized in that** the matrix compounds and/or carriers are selected from the group of collagens, in particular type I collagen and/or type III collagen and/or type IV collagen, scleroproteins, gelatins, chitosans, glucosamines, glycosaminoglycans (GAGs), heparan sulfate proteoglycans, sulfated glycoproteins, growth factors, crosslinked polysaccharides, crosslinked polypeptides and mixtures thereof.

4. The method according to one of the preceding claims, **characterized in that** the at least one three-dimensional sweat gland equivalent provided in method step a) contains at least one cell type selected from the group of (i) coil cells, in particular clear cells, dark cells and myoepithelial cells, (ii) duct cells and (iii) mixtures thereof.

5. The method according to one of the preceding claims, **characterized in that** the at least one secretion protein, in particular the at least one antibacterial secretion protein, is selected in method step b) from the group of mucins, dermcidins, beta-defensins, secretoglobins, lysozyme, albumin, lactate dehydrogenase (LDH), galanin, prolactin-induced protein (PIP), vasoactive intestinal peptide (VIP), zinc-alpha-2-glycoprotein (ZA2G), lipophilin, apolipoproteins, S100 proteins, calcium-dependent activator protein for secretion (CADPS), substance P, calcitonin-gene related peptide (CGRP) and mixtures thereof.

6. The method according to one of the preceding claims, **characterized in that** the identification and analysis in method step b) is carried out using methods selected from the group of molecular biological methods, protein analyses, assays for determining functionality and combinations thereof.

7. The method according to one of the preceding claims, **characterized in that** the influence of at least one compound on the at least one secretion protein identified in method step b) is examined in an additional method step c).

8. The method according to claim 7, **characterized in that** the influence of the at least one compound is examined in method step c) using methods selected from the group of molecular biological methods, protein analyses, assays for determining functionality and combinations thereof.

## Revendications

1. Procédé in vitro permettant l'identification et l'analyse de protéines sécrétoires dans la glande sudoripare humaine, comprenant les étapes de procédé suivantes :
a) mise à disposition d'au moins un équivalent tridimensionnel de glande sudoripare, comprenant de 500 à 500 000 cellules de glande sudoripare, l'au moins un équivalent tridimensionnel de glande sudoripare présentant un diamètre de 100 à 6 000 µm et étant exempt de composés matriciels et de supports, et
b) identification et analyse d'au moins une protéine sécrétoire dans l'au moins un équivalent tridimensionnel de glande sudoripare mis à disposition à l'étape a) du procédé, l'au moins une protéine sécrétoire étant une protéine sécrétoire antibactérienne.

2. Procédé selon la revendication 1, **caractérisé en ce que** l'au moins un équivalent tridimensionnel de glande sudoripare mis à disposition à l'étape a) du procédé présente un diamètre de 100 à 4 000 µm, de préférence de 100 à 2 000 µm, en particulier de 200 à 1 500 µm.

3. Procédé selon la revendication 1 ou 2, **caractérisé en ce que** les composés matriciels et/ou les supports sont choisis dans le groupe des collagènes, en particulier du collagène de type I et/ou de type III et/ou de type IV, des scléroprotéines, des gélatines, des chitosanes, des glucosamines, des glucosaminoglucanes (GAG), des protéoglucanes de sulfate d'héparine, des glycoprotéines sulfatées, des facteurs de croissance, des polysaccharides réticulés, des polypeptides réticulés ainsi que leurs mélanges.

4. Procédé selon l'une des revendications précédentes, **caractérisé en ce que** l'au moins un équivalent tridimensionnel de glande sudoripare mis à disposition à l'étape a) du procédé contient au moins un type de cellule choisi dans le groupe des (i) cellules de queue, en particulier des cellules claires, des cellules sombres ainsi que des cellules de myoépithélium, des (ii) cellules de conduit ainsi que (iii) leurs mélanges.

5. Procédé selon l'une des revendications précédentes, **caractérisé en ce que** l'au moins une protéine sécrétoire, en particulier l'au moins une protéine sécrétoire antibactérienne, est choisie à l'étape b) du procédé dans le groupe des mucines, des dermicidines, des bêta-défensines, des sécrétoglobines, de la lysozyme, de l'albumine, du lactate déshydrogénase (LDH), de la galanine, de la protéine induite par la prolactine (PIP), du peptide intestinal vasoactif (VIP), de la zinc-alpha-2-glycoprotéine (ZA2G), de la lipophiline, des apolipoprotéines, des protéines S100, de la protéine activatrice de sécrétion dépendante du calcium (CADPS), de la substance P, du peptide apparenté au gène de la calcitonine (CGRP) ainsi que leurs mélanges.

6. Procédé selon l'une des revendications précédentes, **caractérisé en ce que** l'identification et l'analyse sont effectuées à l'étape b) du procédé à l'aide de méthodes choisies dans le groupe des méthodes de biologie moléculaire, des analyses de protéines, des dosages destinés à déterminer la fonctionnalité et leurs combinaisons.

7. Procédé selon l'une des revendications précédentes, **caractérisé en ce que** l'influence d'au moins un composé sur l'au moins une protéine sécrétoire identifiée à l'étape b) du procédé est étudiée dans une étape supplémentaire c) du procédé.

8. Procédé selon la revendication 7, **caractérisé en ce que** l'influence de l'au moins un composé à l'étape c) du procédé est effectuée à l'aide de méthodes choisies dans le groupe des méthodes de biologie moléculaire, des analyses de protéines, des dosages destinés à déterminer la fonctionnalité et leurs combinaisons.
